# EUROPEAN PATENT APPLICATION

(11) **EP 1 920 734 A2**
(43) Date of publication of application: **14.05.2008**
(21) Application number: 07021645.2
(22) Date of filing: 07.11.2007
(51) Int. Cl.: A61F 2/06, A61F 2/84

(54) **Modular stent graft and delivery system**

(30) Priority: 09.11.2006 US 595282
(71) Applicant: Chui's Hong Kong Invention Promotion Ltd., 10 Des Voeux Road Central Hong Kong (CN)
(72) Inventor: Yu, Chun-Ho, Hong Kong (CN)
(74) Representative: Dossmann, Gérard

(57) **Abstract**

A modular aortic stent graft and a modular branched stent graft, expandable between a compressed condition and an expanded condition, for assembling with each other at the treatment area of an aneurysm. The aortic stent graft includes at least one axial opening for receiving a branched segment of the branched stent graft. The branched stent graft includes a branched segment extending from an aortic segment thereof. The aortic segment of the branched stent graft is disposed inside the aortic stent graft, while the branched segment is disposed through the opening of the aortic stent graft in the expanded condition. A set of aortic stent grafts and branched stent grafts are provided in various dimensions to cater the needs for different patients.

## Description

This invention relates to a medical device, in particular, a modular stent graft and a system for delivering modular stent graft to a target location in a blood vessel for the treatment of an aneurysm.

An aneurysm is a weak area in an artery, which may bulge and enlarge due to the blood pressure over time. An aneurysm often affects the aorta, the largest artery that carries blood from the heart through chest and abdomen. An aneurysm may result in rupture, causing massive internal bleeding, and may be fatal if not being treated immediately.

A conventional way to treat an aneurysm is to place a stent graft in the aorta at the location of the aneurysm. A stent graft is an expandable wired framework supporting a continuous sealed conduit which opens at opposite ends for blood to flow through. The stent graft seals the aneurysm and allows blood to flow through without adding pressure on the bulge. The stent graft is usually placed with a delivery sheath and opens by a self-expanding mechanism.

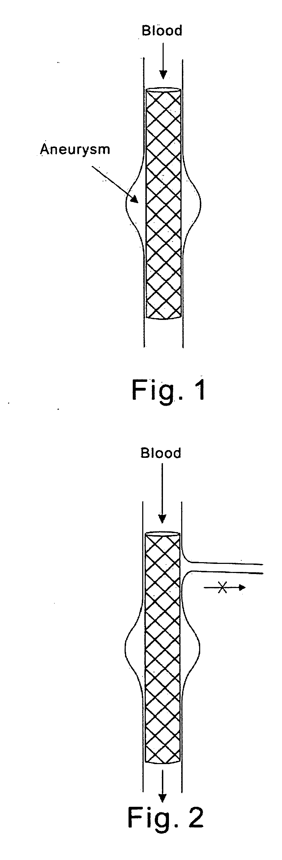

As shown in Fig. 1, the stent graft has a distal portion and a proximal portion in contact with the vessel wall, at least 2 cm immediately above and below the aneurysm for supporting the stent graft. Such portions require a minimal length of 2 cm to provide an effective and reliable sealing zone to prevent leaking of blood into the aneurysm causing further expansion of the aneurysm.

As shown in Fig. 2, a problem is created when the aneurysm is located close to one or more aortic branches. In that case, the distal or the proximal portion of the stent graft may be located at the bisection of the aorta and the aortic branch and block the blood flow to the aortic branch. In some cases, more than one aortic branch is blocked.

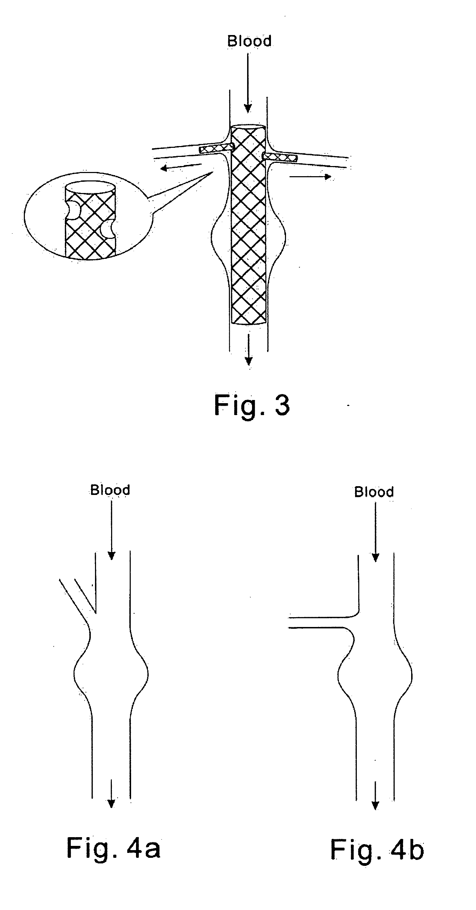

Currently, as shown in Fig. 3, fenestrated aortic stent grafts are used in treating aneurysms at such location. A fenestrated aortic stent graft is tailor-made for the individual patient to provide openings on the stent graft to allow blood to flow through the openings into the aortic branches. The position of the openings must be accurate to fit with the aortic branches. A separate small branched stent graft is placed at the aortic branch near the bisection, for guiding the blood to flow from the fenestrated aortic stent graft into the aortic branch. The branched stent graft also provides a seal to prevent leaking of blood into the aneurysm causing further expansion of the aneurysm.

The disadvantage of the above treatment method is the customization of such fenestrated aortic stent graft requires an accurate imaging of the aorta and the aortic branch of the patient in order to position the openings on the stent graft. The preparation and manufacturing of a fenestrated aortic stent graft take weeks to accomplish. In an emergency case, a burst aorta is fatal. There is no time to custom-make a fenestrated aortic stent graft to deal with such an urgent situation. The placement of fenestrated aortic stent graft also requires a high level of expertise of the surgeon in handling the tedious procedural steps.

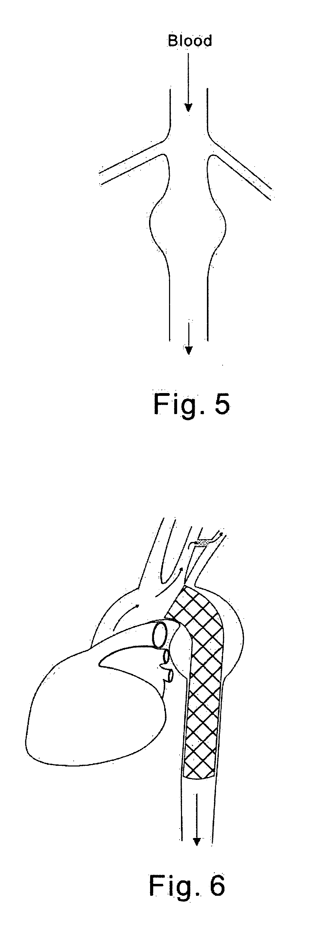

Another disadvantage of the above treatment method is that it is not effective if the treatment area is at the downwardly pointing arterial branches such as the renal artery and the superior mesenteric artery. The existing delivery system of the branched stent graft is capable of delivering stent graft to the upwardly pointing arterial branches or arterial branches which are substantially perpendicular to the aorta as shown in Figs. 4a and 4b. However, the existing delivery system introduced from below is not flexible enough to turn backward to enter into the acutely downward-pointing arterial branches such as those shown in Fig. 5. At such treatment area, a guiding catheter is required to be inserted from another entry site from above to get through the stent graft and enter into the arterial branch, in order to place a covered stent into the arterial branch from above to connect with the stent graft.

In some treatment areas, multiple entry sites are required if more than one branched stent graft is involved. The delivery procedure is therefore complicated. If such entry is required at the neck vessels, there is a risk in causing complications to the patient's brain.

Another conventional way of treatment, as shown in Fig. 6, is before placing an aortic stent graft which will block the aortic branch, a bypass surgery is performed so that the blood can be re-routed to the affected aortic branch from another aortic branch nearby. Sometimes a few aortic branches are blocked by an aortic stent graft, and more than one bypass is necessary. Such bypass surgery can only be conducted in certain hospitals which provide the specific instruments and apparatus. In an emergency, the patient may not have time to be transferred to those hospitals to undergo the bypass surgery. Such bypass surgery also increases the medical risk on the patient and substantially prolongs the procedural time of treatment.

Therefore, there is a need for a set of ready-made modular stent grafts which deal with situation when the aneurysm is near to a bisection of aorta and aortic branch, and is applicable to any individual patient. There is also a need for a delivery system for placing the modular stent graft, especially the branched stent graft, in a convenient and effective manner. In addition, there is a need for a delivery system of a branched stent graft for target location at downwardly pointing arterial branch which requires only one entry site using much simplified delivery procedure.

An object of the present invention is to provide a set of modular stent grafts for a more effective treatment of an aneurysm at a location near to a bisection of aorta and aortic branch.

An embodiment of the present invention is a modular aortic stent graft and a modular branched stent graft for assembling with each other.

Another embodiment of the present invention is a set of modular aortic stent grafts each having at least one axial opening, such set having variations in length and diameter of stent graft, and in number, size and position of the axial opening thereon, readily applicable to any individual patient.

Another embodiment of the present invention is a modular set of branched stent grafts each having an aortic segment and a branched segment, such set having variations in length and diameter of the aortic segment and branched segment, readily applicable to any individual patient.

Another embodiment of the present invention is a modular aortic stent graft with at least one axial opening, and a modular branched stent graft to be placed in the aortic stent graft, with a branched segment inserting through the axial opening, while the rest of the opening being covered by the aortic segment of the branched stent graft.

Another embodiment of the present invention is a modular branched stent graft for associating with another modular branched stent, such that a proximal portion of the distal branched stent graft is disposed in a distal portion of the proximal branched stent graft.

Another embodiment of the present invention is a modular branched stent graft partially covered by a graft layer having a non-grafted portion such that when the branched stent graft is assembled with an aortic stent graft having more than one opening, the other openings are not blocked by the graft layer of the branched stent graft, and the branched stent graft can be further assembled with other branched stent grafts for blood and the branched segments to pass through.

Another embodiment of the present invention is a modular aortic stent graft and a modular branched stent graft with radiopaque markings for indicating the orientation and position of the stent graft inside human body.

Another embodiment of the present invention is a stent graft delivery system for delivering a branched stent graft into an aortic stent graft and placing a branched segment of the branched stent graft at a target location in an aortic branch.

Another embodiment of the present invention is a stent graft delivery system for which only one entry site is required for delivering the branched stent graft at a target location of a downwardly pointing arterial branch.

Another embodiment of the present invention is a stent graft delivery system including an engaging member having an axially reversed portion disposed in a carrier member in a pre-loaded condition, such reversed portion is engaged with a branched segment of a branched stent graft in the pre-loaded condition.

Another embodiment of the present invention is a stent graft delivery system including an engaging member having an axially reversed portion for delivering a branched segment of a branched stent graft into an aortic branch in a backward manner.

Another embodiment of the present invention is a stent graft delivery system including a guiding member having an axially reversed portion partially disposed in a reversed portion of an engaging member, and partially disposed outside the engaging member through a hole in a turning portion thereof in a pre-loaded condition, such guiding member guides the engaging member engaged with a branched segment of a branched stent graft into an aortic branch.

Another embodiment of the present invention is a stent graft delivery system including a guiding member having an axially reversed portion for guiding an engaging member engaged with a branched segment of a branched stent graft into an aortic branch in a backward manner.

Another embodiment of the present invention is a stent graft delivery system including a middle core for maintaining the position of a branched stent graft during the withdrawal of a carrier member in releasing a reversed portion of an engaging member.

The above and other aspects, features, and advantages of the present invention will become more apparent upon consideration of the following detailed description of preferred embodiments, taken in conjunction with the accompanying drawing figures, wherein:-
Fig. 1 shows a conventional stent graft used in the current treatment of an aneurysm;
Fig. 2 illustrates a problem in using the conventional stent graft of Fig. 1 in the current treatment of an aneurysm;
Fig. 3 shows conventional fenestrated aortic stent grafts used in the current treatment of an aneurysm in location near aortic branch;
Fig. 4a illustrates a treatment area at an upwardly pointing arterial branch;
Fig. 4b illustrates a treatment at an arterial branch perpendicular to an aorta;
Fig. 5 illustrates a treatment area at a downwardly pointing arterial branch;
Fig. 6 illustrates a bypass surgery for treating an aneurysm in location near an aortic branch;
Fig. 7 is a perspective view of an aortic stent graft and a branched stent graft before and after being assembled in accordance with an embodiment of the present invention;
Fig. 8 is a perspective view of the aortic stent graft of Fig. 7;
Figs 9a and 9b are a perspective view and a top view of the aortic stent graft of Fig. 7;
Fig. 10 is a perspective view of a branched stent graft of Fig. 7;
Fig. 11 illustrates the use of the aortic stent graft and the branched stent graft of Fig. 7 at a treatment area;
Fig. 12 illustrates the use of an aortic stent graft in accordance with an embodiment of the present invention at a treatment area with more than one aortic branch;
Fig. 13 is a perspective view of an aortic graft and three stent branch grafts being assembled in accordance with an embodiment of the present invention;
Fig. 14 is a cross-section view of Fig. 13 along line b-b;
Fig. 15 is a perspective view of two branched stent grafts in accordance with an embodiment of the present invention;
Fig. 16 is a sectional side view of a delivery system for delivering an aortic stent graft of Fig. 7 in the pre-loaded condition;
Fig. 17 is a top view of a straight delivery sheath for delivering an aortic stent graft of Fig. 7;
Fig. 18 is a side view of a J-shaped delivery sheath for delivering an aortic stent graft of Fig. 7;
Fig. 19 is a sectional side view of a delivery system of an embodiment in the pre-loaded condition in accordance with an embodiment of the present invention;
Fig. 20 is a partial sectional side view of a guiding member and an engaging member of the delivery system of Fig. 19 in the pre-loaded condition.
Fig. 21 is a partial sectional side view of a guiding member and an engaging member of the delivery system of Fig. 19 loaded with a branched stent graft in the pre-loaded condition.
Fig. 22a is a perspective view of a branched stent graft in the compressed condition loaded in the delivery system of Fig. 19 without showing the engaging member;
Fig. 22b is a perspective view of a branched stent graft in the compressed condition loaded in the delivery system of Fig. 19 also showing the engaging member;
Fig. 23 is an enlarged cross-section view of Fig. 19 along line x-x;
Fig. 24 is an enlarged cross-section view of Fig. 19 along line y-y;
Figs. 25 and 26 illustrate the folding of the branched stent graft of Fig. 7 from the expanded condition to the compressed condition;
Fig. 27a is a sectional side view of a delivery system in the pre-loaded condition in accordance with an embodiment of the present invention;
Fig. 27b is an enlarged cross-section view of Fig. 27a along line b-b;
Figs. 28a - 28f illustrate the steps in using a delivery system in accordance with an embodiment of the present invention;
Figs. 29a - 29e are the front views of the delivery system in accordance to the steps illustrated in Figs. 28a - 28f; and
Figs. 30a and 30b are flow diagrams illustrating the use of the delivery system in accordance with the steps illustrated in Figs. 28a - 28f.

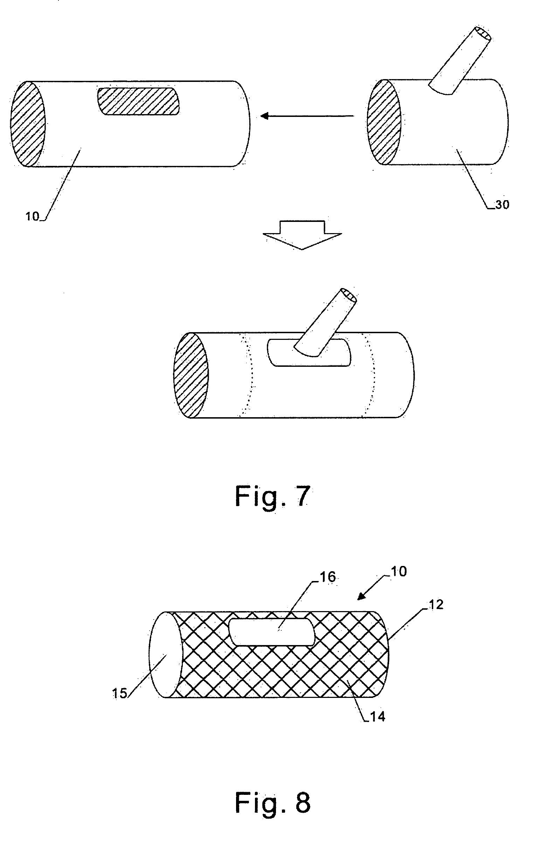

As illustrated in Fig. 7, a preferred embodiment of the present invention includes an aortic stent graft [10] and a branched stent graft [30], which can be assembled together for the treatment of an aneurysm in the aorta at location near an aortic branch.

### Aortic stent graft

As illustrated in Fig. 8, the aortic stent graft [10] may be a conventional stent graft formed by a wired framework known as stent [12] and a graft layer [14]. The stent [12] is usually made of stainless steel or an alloy such as nickel-titanium or nitinol. The graft layer [14] is made of a non-porous and durable material such as polytetrafluoroethylene (PTFE), or knitted or woven mono filament polyester fabric, forming a conduit with a lumen [15] to keep the blood from applying pressure on the affected portion of the vessel. The stent [12] is expandable between a compressed condition, in which it can be delivered to the desired location for treatment, and an expanded condition for opening up and supporting the graft layer [14] in the vessel. Before the stent [12] is fully open, there may be a semi-expanded condition for the surgeon to adjust the position of the aortic stent graft [10] inside the aorta.

The aortic stent graft [10] may be self-expandable or to be expanded by an internal radial force, such as an angioplastry balloon. By way of example, the self-expandable aortic stent graft [10] is used in the following embodiments.

The aortic stent graft [10] further contains an opening [16] on the graft layer [14] and the stent [12] along the axis for allowing blood to flow from the aorta to an aortic branch at the treatment area. The size of opening [16] does not have to be custom-made to fit the size of the lumen of the arterial branch of each individual patient. The size of the opening [16] may be much bigger than the lumen of the arterial branch for accommodating more than one branched stent graft if necessary. The extra opening space will be covered by the aortic segment of the branched stent graft to be assembled with the aortic stent graft [10], which will be further explained below.

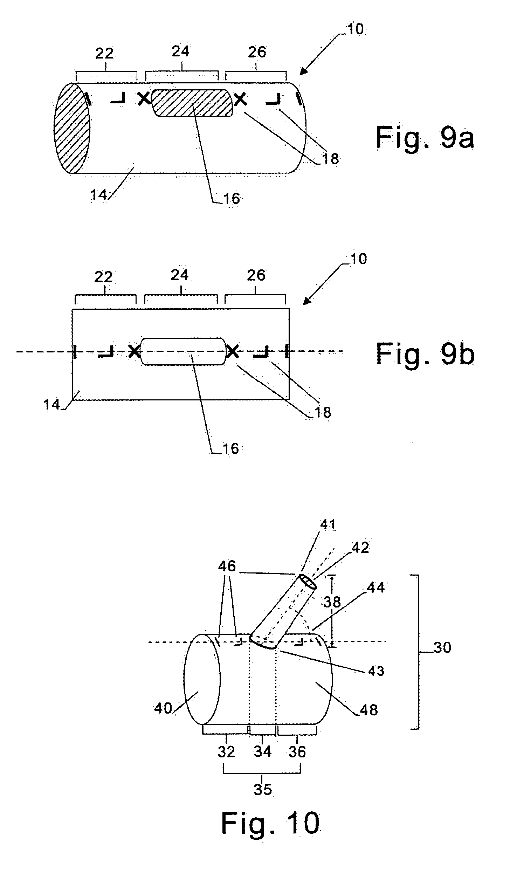

As illustrated in Figs. 9a and 9b, the aortic stent graft [10] is divided into a distal portion [22], a middle portion [24] between the endings of the opening [16], and a proximal portion [26]. Radiopaque markings [18] which are visible under imaging equipment are marked on the graft layer [14] along the axis of the opening [16] to indicate the orientation and the position of the aortic stent graft [10] and the opening [16] inside human body. By way of example, the markings [18] are marked at the two edges of the opening [16], the middle parts of the distal portion [22] and the proximal portion [26], and the two ends of the aortic stent graft [10], all along the axis of the opening [16]. In order to show the orientation of the opening [16], by way of example, the alphabet "L" is used for the markings [18]. If a reversed "L" is shown under the imaging instrument, the opening [18] is on the opposite side and has to be rotated to the correct orientation. An example of the radiopaque material for the markings [18] is gold.

The aortic stent graft [10] may be a straight or curved body, depending on the location of the treatment area. A set of the aortic stent grafts [10] are provided with variations in the size of the distal portion [22], the proximal portion [26] and the opening [16] to deal with different locations of treatment area and cater for the needs of different patients. By way of example, the diameter of the distal portion [22] may range from about 2 cm to about 5 cm, the diameter of the proximal portion [26] about 2 cm to about 4 cm. The length of the distal portion [22] may range from about 2 cm to about 3 cm and the proximal portion [26] may range from about 3 cm to about 10 cm. The length of the opening [26] may range from about 2 cm to about 10 cm, depending on the number of the branched stent graft [30] to be assembled with the aortic stent graft [10].

There may be a scallop member disposed near the distal end of the distal portion [22] to accommodate an adjacent aortic branch so that it is not blocked by the distal portion [22]. The proximal end of the proximal portion [26] may further include bifurcated stent grafts that extend into the common iliac arteries.

### Branched stent graft

The branched stent graft is also formed by a wired framework known as stent and a graft layer as found in a conventional stent graft. The stent is usually made of stainless steel or an alloy such as nickel-titanium or nitinol. The graft layer is made of a non-porous and durable material such as polytetrafluoroethylene (PTFE) or knitted or woven mono filament polyester fabric. The branched stent graft may be self-expandable or to be expanded by an internal radial force, such as an angioplasty balloon. By way of example, the self-expandable branched stent graft is used in the following embodiments.

As illustrated in Fig. 10, the branched stent graft [30] includes a branched segment [38] and an aortic segment [35]. The aortic segment [35] contains a distal portion [32], a proximal portion [36], a middle portion [34] from where the branched segment [38] extends and a main lumen [40]. The branched segment [38] contains an open end [41], a connecting end [43] in connection with the middle portion [34] of the aortic segment [35] and a branch lumen [42]. The branch lumen [42] of the branched segment [38] connects with the main lumen [40] so that the blood may flow from the middle portion [34] into the branched segment [38] and into an aortic branch. The axis of the branched segment [38] forms an adjacent angle [44] ranging from about 40 degrees to about 80 degrees with the axis of the middle portion [34]. By way of example, the adjacent angle [44] in the preferred embodiment is about 60 degrees.

Radiopaque markings [46] are marked on the graft layer [48] along the axis of the aortic segment [35] and the branched segment [38] to indicate the orientation and the position of branched stent graft [30], especially the branched segment [38]. By way of example, the markings [46] are marked at the two ends of the aortic segment [35], the middle parts of the distal and proximal portions, and the open end [41] and the connecting end [43] of the branched segment [38].

The aortic segment [35] may form a straight or curved body, depending on the location of the treatment area. A set of the branched stent grafts [10] are provided with variations in the size of the distal, proximal and branched segments [32, 36, 38] to deal with different locations of treatment area and cater for the needs of different patients. By way of example, the diameter of the distal portion [32] may range from about 1 cm to about 5 cm, the diameter of the proximal portion [36] about 1 cm to about 4 cm, the diameter of the branched segment [38] about 0.5 cm to about 2 cm. The length of the distal portion [32] may range from about 1 cm to about 2 cm, the length of the proximal portion [36] about 1 cm to about 2 cm and the length of the branched segment [38] about 2 cm to about 3.5 cm.

Each of the distal portion [32] and the proximal portion [36] has the same diameter along the respective portion. The diameter of the proximal portion [32] of the aortic segment [35] may be slightly smaller than the diameter of the distal portion [36]. Such arrangement allows the proximal portion of the distal branched stent graft to be able to fit in the distal portion of the proximal branched stent graft when two branched stent graft are assembled together, which will be further discussed below.

The branched stent graft [30] may operate between a compressed condition and an expanded condition. As known in the art, a stent graft is wrapped and tied by a holding wire in the compressed condition before deployment. A holding wire laces through the wired framework of a stent graft and is sewn to the grafted layer to temporarily hold the stent graft in a compressed condition. The holding wire is usually kept in place by a knot or a crook such that the holding wire may be released by pulling the holding wire from the outside to release the stent graft. The use of a holding wire in temporarily holding a stent in a compressed condition is disclosed in US Patent No. 5,443,500 and US Patent Publication No. US 2001/0005793 A1, which are incorporated by reference.

In the preferred embodiment, the aortic segment [35] is folded and packed into the compressed condition by a first holding wire (not shown) and a semi-expanded condition by a second holding wire (not shown). The aortic segment [35] is released to the expanded condition by pulling and untying the first and second holding wires respectively. The branched segment [38] is folded and packed into the compressed condition by a third holding wire (not shown), which can be released to the expanded condition by pulling and untying the third holding wire.

### Assembly of aortic and branched stent grafts

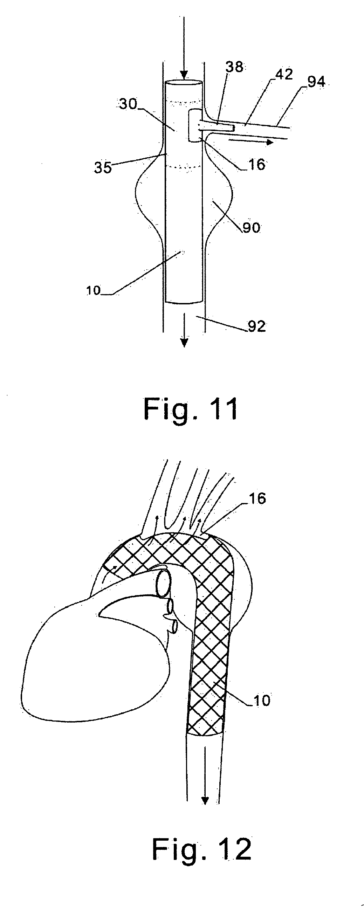

As illustrated in Fig. 11, at the treatment area where the aneurysm [90] is near to an aortic branch [94], the opening [16] is positioned to superimpose with the lumen of the aortic branch [94] so that the aortic stent graft [10] will not block the blood from flowing to the aortic branch [94]. The branched stent graft [30] is then disposed in the aortic stent graft [10] so that the branched segment [38] extends through the opening [16] into the aortic branch [94]. The chosen branched stent graft [30] should have the diameter of the aortic segment [35] conformed with the diameter of the aortic stent graft [10].

In expanding the branched stent graft [30], the aortic segment [35] is first deployed to the semi-expanded condition by pulling and untying the first holding wire. In this condition, the branched segment [38] may be adjusted to a more accurate position inside the aortic branch [94]. After that, the aortic segment [35] is deployed to the fully expanded condition by pulling and untying the second holding wire. Finally, the third holding wire is pulled and untied to deploy the branched segment [38] to the expanded condition.

In treatment area where there exists more than one aortic branch, as illustrated in Fig. 12, more than one branched stent graft [30] may be required to be assembled with the aortic stent graft [10]. In the case as shown in Fig. 12, the aortic stent graft [10] with an opening [16] long enough to superimpose with the lumen of three aortic branches is selected.

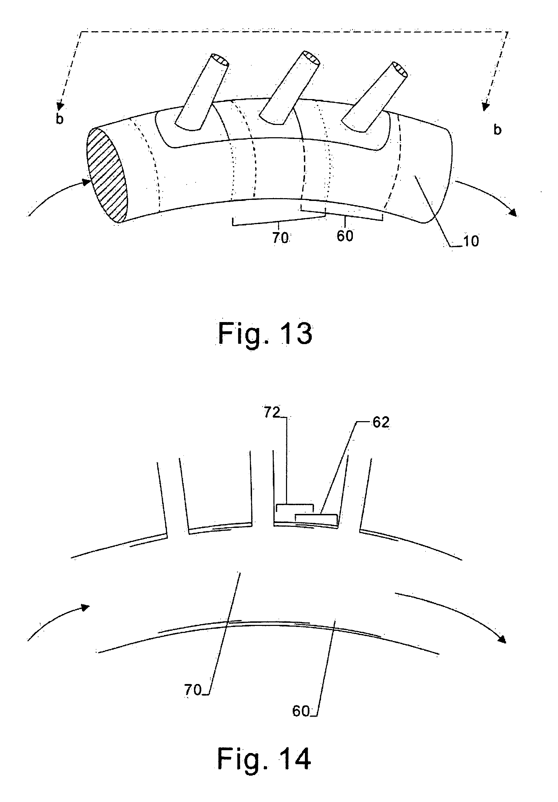

The distal and proximal portions of the aortic segment [35] of the branched stent graft [30] are configured and sized to assemble with the corresponding portion of the aortic segment of another branched stent graft. As illustrated in Figs. 13 and 14, the branched stent grafts are disposed in the aortic stent graft [10] against the direction of the blood flow. A proximal branched stent graft [60] is disposed first, followed by another branched stent graft [70] disposed distal to the branched stent graft [60]. The proximal portion [72] of the distal branched stent graft [70] is partially disposed in the distal portion [62] of the proximal branched stent graft [60]. Such arrangement ensures that the blood flow is not interrupted at the overlapping portions of the branched stent grafts. However, the order of stent graft placement may be varied depending on the circumstances of each case.

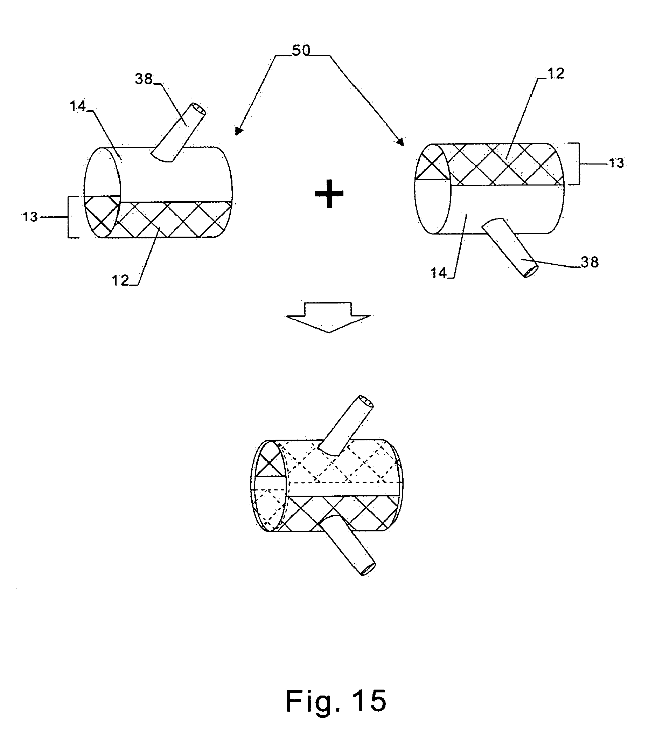

In some treatment areas, there exists aortic branches on both sides of the aorta. The aortic stent graft needs to have, for example, at least one opening generally opposed from another opening. In that case, as illustrated in Fig. 15, a branched stent graft [50] only partially covered by the graft layer [14] on the side of the branched segment [38] is provided to allow a branched segment of another associated branched stent graft or blood to pass through a non-grafted portion [13] of the branched stent graft [50] into the aortic branch on the opposite side. The two partially grafted stent grafts [50] are disposed in an aortic stent graft having two openings each for the corresponding branched segment [38].

### Delivery system of aortic stent graft

The aortic stent graft [10] may be deployed to the aorta at the treatment area by the conventional endovascular stent graft placement procedure using a conventional delivery system [100] including a delivery sheath [101], an inner core [108] and a middle core [116].

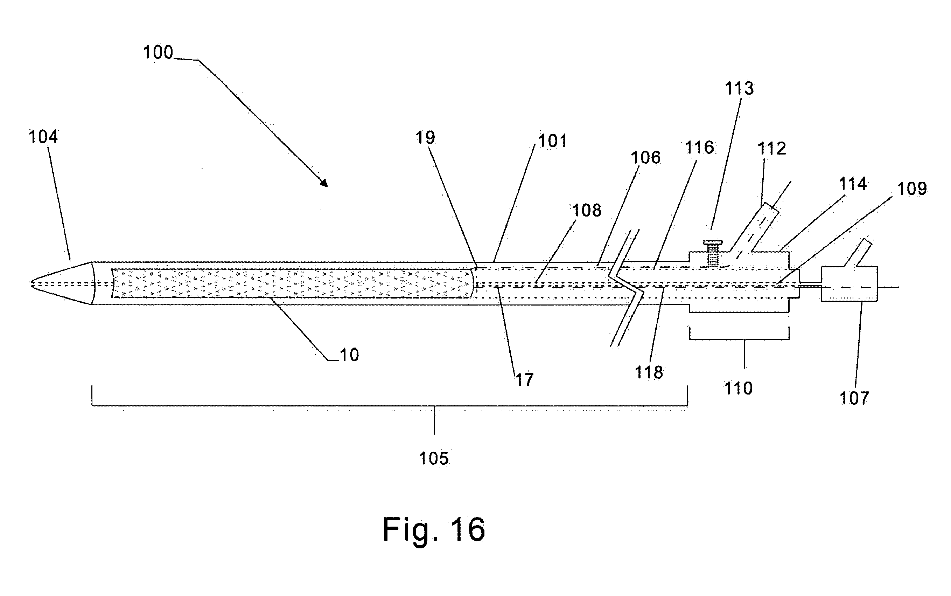

As illustrated in Fig. 16, the delivery sheath [101] further includes a flexible tapered tip [104] at the distal end and a sheath portion [105] with hydrophilic coating for containing the aortic stent graft [10] in the sheath lumen [106] near the distal end. At the proximal end of the delivery sheath [100], there is a hub [110] including a flush port [112] for irrigation to and for expelling air out of the sheath lumen [106], as well as a hemostatic valve [114] for preventing leakage of the body fluid.

An inner core [108] extends from the tip [104] throughout the sheath lumen [106] and comes out from the hub [110]. The inner core [108] includes a central lumen [109] for receiving a guide wire which guides the delivery system [100] to the target treatment area. The inner core [108] further includes a hub [107] at the proximal end for irrigating the central lumen of the inner core [108].

The aortic stent graft [10] is first tied up by a holding wire [17] into a semi-compressed condition, which is then further folded and packed into a compressed condition in a manner known in the art before being pre-loaded at the distal end of the sheath lumen [106].

A middle core [116] is disposed in the sheath lumen [106] terminating at the aortic stent graft [10]. The middle core [116] contains a central lumen [118] for receiving the inner core [108]. The aortic stent graft [10] is attached to the delivery system [100] by a locking mechanism engaging a locking wire in a manner known in the art, which allows the aortic stent graft [10] to be moved and rotated with the delivery system [100] during the deployment. The locking wire may be a metal wire with a distal end removably coupled to a strut disposed at the proximal end of a stent graft and attached to the distal end of a middle core or an inner core. The use of a locking wire in locking a stent graft to a delivery system is disclosed in US Patent Nos. 6,761,733, 5,201,757 and US Patent Publication No. US 2006/0004433 A1, which are incorporated by reference.

By way of example, the locking wire [19] is removably coupled to the proximal end of the aortic stent graft [10] and engaged to the distal end of the middle core [116]. The locking wire [19] further extends out the delivery system [100] along with the middle core [116]. A screw lock [113] may be provided at the proximal end of the middle core [116] to secure the locking wire [19] on the middle core. The aortic stent graft [10] may be detached from the delivery system [100] by unscrewing the screw lock [113] and pulling the locking wire [19].

The delivery sheath [101] may be a straight sheath for treatment at the abdominal aorta, of L-shape for descending thoracic aorta, or of J-shape for arch and ascending thoracic aorta.

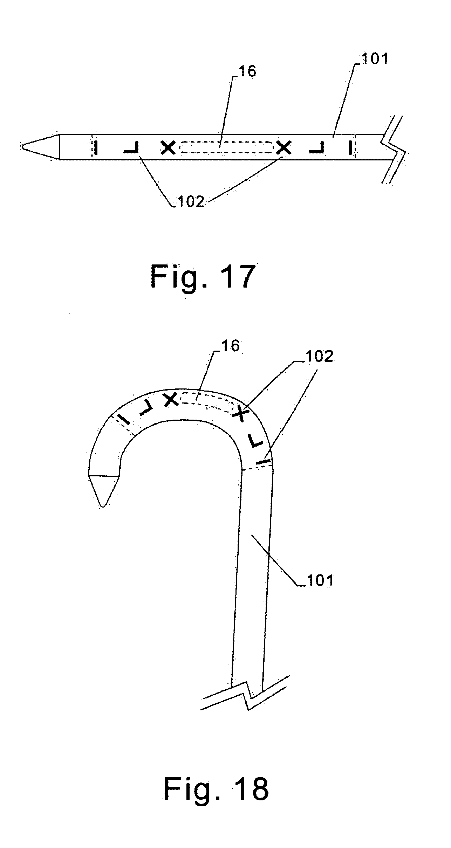

In the compressed condition, the aortic graft [10] of Figs. 9a and 9b is folded in a way such that the markings [18] appear at the outer surface to indicate the orientation of the opening [16] when being loaded in the delivery sheath [101]. As shown in Fig. 17, markings [102] indicating the orientation and position of the opening [16] are also marked outside of the straight delivery sheath [101] to assist the surgeon to insert the delivery sheath [101] into the patient's body with the opening [16] in the correct orientation.

As illustrated in Fig. 18, for the L-shaped or J-shaped delivery sheath, the markings [102] should appear at the outer curved surface to match with the location of the opening [16] for treatment at the corresponding area.

### Delivery of aortic stent graft

The target aortic area to be stented and the aortic branch affected are first identified on the aortogram. The aortic stent graft [10] is to be positioned at the treatment area such that the opening [16] is superimposed with the lumen of the aortic branch. The aortic stent graft [10] may be delivered to the target location by an endovascular stent graft placement procedure.

By way of example, a guide wire is first inserted into the patient's body to guide the delivery system [100] of Fig. 16 to the target treatment area. The delivery system [100] pre-loaded with the aortic stent graft [10] is then inserted into the aorta and follows the guide wire until the aortic stent graft [10] has reached the target location. The position of the aortic stent graft [10] and the orientation of the opening [16] are indicated on an imaging instrument by the markings [18]. The aortic stent graft [10] is adjusted to a location such that the opening [16] is superimposed with the lumen of the aortic branch. The aortic stent graft [10] is then released from the delivery sheath [100] by slidably pulling the sheath portion [105] backward, which releases the aortic stent graft [10] to the semi-expanded condition.

After the opening [16] of the aortic stent graft [10] has been well aligned with the lumen of the aortic branch by rotating the middle core [116] which is attached to the aortic stent graft [10], and the position has been confirmed by checking the markings [18] adjacent to the opening [16], the holding wire [17] of the aortic stent graft [10] is pulled backward to un-tie the aortic stent graft [10] so that it expands from the semi-expanded condition to the fully expanded condition.

The locking wire [19] is then slidably withdrawn to detach the aortic stent graft [10] from the middle core [116]. At this position, the opening [16] is superimposed with the lumen of the aortic branch ready to receive the branched segment [38] of a branched stent graft [50]. The delivery system [100] can then be withdrawn from the patient's body.

### Delivery system of branched stent graft

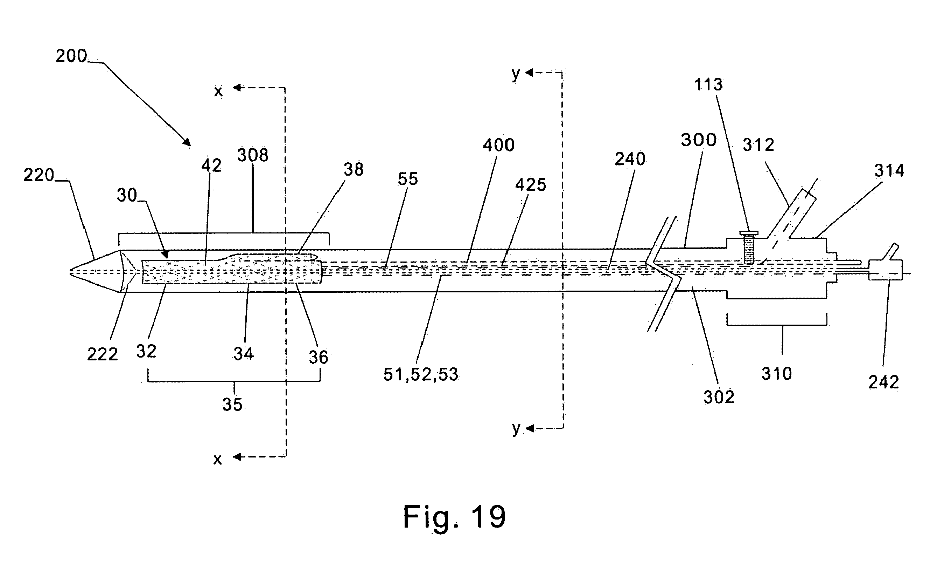

A preferred embodiment of this invention is used to deliver a branched stent graft to be assembled with an aortic stent graft inside the patient's body. As illustrated in Figs. 19, 23 and 24, a delivery system [200] contains a tapered tip [220], an inner core [240], a carrier member [300] (for example, an outer sheath), an engaging member [400] (for example, a branch catheter), and a guiding member [500] (for example, a branch wire).

### Carrier Member

The elongated carrier member [300] carries and delivers the engaging member [400] loaded with the branched stent graft [30] to a target location near the treatment area. The carrier member [300] may be a conventional outer sheath having a lumen [302] with hydrophilic coating for containing the branched stent graft [30] in a stent graft portion [308] at the distal end. The size and the configuration of the carrier member [300] vary according to the target location of the branched stent graft. A straight carrier member is applicable for the abdominal aorta and a J-shaped carrier member for the arch and ascending thoracic aorta. By way of example, the lumen [302] may be of an inner diameter ranging from about 3 mm to about 8 mm.

At the proximal end of the carrier member [300], there is a hub [310] including a flush port [312] for irrigation to and for expelling air out of the lumen [302], as well as a hemostatic valve [314] for preventing leakage of body fluid.

### Inner core

The inner core [240] extends from the distal end of the carrier member [300] along the lumen [302] and comes out from the hub [310]. The distal end of the inner core [240] is attached to the tip [220] which is releasably connected to the distal end of the carrier member [300]. The tapered shape of the tip [220] makes it easier for the carrier member [300] to be inserted from outside the patient's body and move forward in the aorta. The tip [220] has a back portion [222] which tapers in the proximal direction. The back portion [222] facilitates the withdrawal the tip [220] at the turns along the aorta. By way of example, the tip [220] is about 2 cm long and the inner core [240] has a diameter of about 0.09 cm.

### Engaging Member

The elongated engaging member [400] is for engaging with the branched stent graft [30] in the compressed condition and delivering the branched segment [38] of the branched stent graft into an aortic branch in a backward manner. The engaging member [400] is pre-loaded in the carrier member [300] with an axially reversed portion at the distal end pointing in the proximal direction. The reversed portion is engaged with the branched segment [38] of the branched stent graft [30] and is disposed at the distal portion of the carrier member [300]. Such arrangement facilitates the deployment of the branched segment [38] by the engaging member [400] in a backward manner, especially if the targeted aortic branch is in a downwardly pointing direction as shown in Fig. 5.

The engaging member [400] is disposed axially along the lumen [302] of the carrier member [300] parallel with the inner core [240] and extends out from the hemostatic valve [314] of the hub [310] of the carrier member [300]. The engaging member [400] having a lumen may further contain the guiding member [500] for guiding the engaging member into the aortic branch. The engaging member [400] may be a conventional catheter, such as a 4 French catheter. A lumen [425] of a diameter, for example, about 0.09 cm runs through the whole engaging member.

As illustrated in Figs. 19, 20, 23 and 24, in the pre-loaded condition, there is an axially reversed portion [410] of the engaging member [400] following the tip [440], which folds backward in the proximal direction. A turning portion [430] follows the reversed portion [410] having a convex side [431] and a concave side [432], followed by an aortic portion [420] which extends out along the delivery system [200]. A hole [435] is formed at the convex side [431] of the turning portion [430] on the engaging member [400] for the guiding member [500] to pass through, which will be further described below.

A flexible tapered tip [440] is formed at the end of the engaging member [400] and tapers to an opening of the lumen [425] for the guiding member [500] to come out in the released condition. The tapered shape of the tip [440] makes it easier for the engaging member [400] to proceed forward in an aortic branch. A back portion [442] of the tip [440] tapers in the opposite direction with a streamlined oval configuration to ensure that during the withdrawal of the engaging member [400] from the aortic branch, the back portion [442] of the tip [440] will not be caught at the bi-section corner of the aorta and the aortic branch, and can be withdrawn smoothly from the aortic branch.

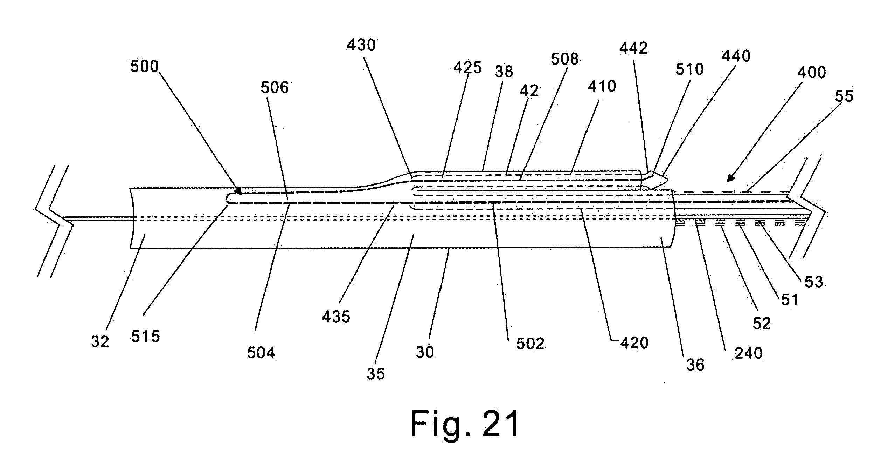

As illustrated in Fig. 21, in the pre-loaded condition, the engaging member [400] is engaged with the branched stent graft [30] in the compressed condition. The reversed portion [410] of the engaging member [400] runs through the branch lumen [42] of the branched segment [38] of the branched stent graft [30], and the aortic portion [420] of the engaging member runs through the main lumen [40] of the aortic segment [35] of the branched stent graft. In the compressed condition, the branched stent graft [30] is tied up by holding wires [51, 52, 53].

The aortic segment [35] of the branched stent graft [30] is tied up by the first holding wire [51] in the semi-expanded condition and the second holding wire [52] in the compressed condition. The branched segment [38] is tied up by the third holding wire [53] in the compressed condition.

The reversed portion [410] and the aortic portion [420] of the engaging member [400] loaded with the branched stent graft [30] are disposed in the lumen [302] at the distal end of the carrier member [300].
The branched stent graft [30] is further attached to the delivery system [200] by a locking wire [55]. The proximal end of the aortic segment [35] is removably coupled to the delivery system [200] by a locking mechanism engaging the locking wire [55] in a manner known in the art, which allows the aortic stent graft [10] to be moved and rotated with the delivery system [200] during the deployment.

By way of example, the branched stent graft [30] is attached to the distal end of the inner core [240] by the locking wire [55]. The locking wire [55] further extends out the delivery system [100] along with the inner core [240]. A screw lock [113] may be provided at the proximal end of the inner core [240] to lock the locking wire [55] on the inner core. The branched stent graft [50] may be detached from the delivery system [200] by unscrewing the screw lock [113] and pulling the locking wire [55].

### The folding of branched stent graft

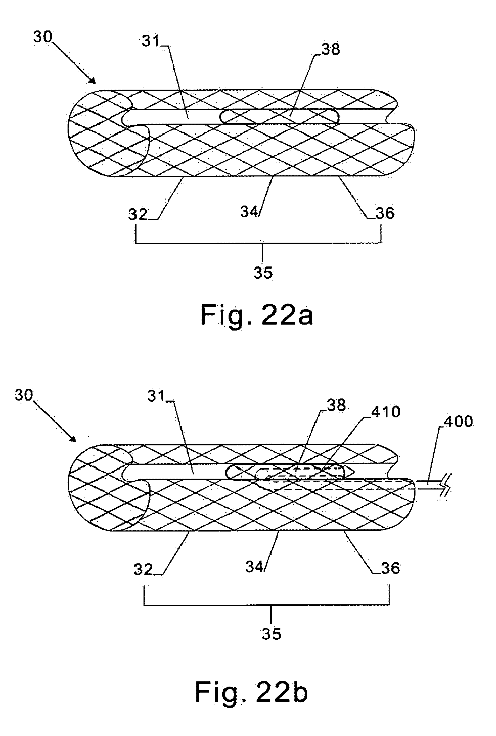

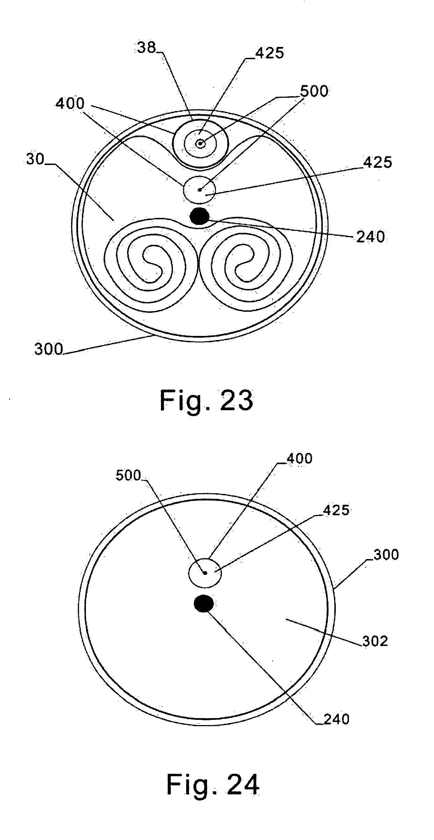

As illustrated in Figs. 22a, 22b and 23, in order to make the branched stent graft [30] more compact in the compressed condition, the aortic segment [35] may be folded into an indented cylindrical shape leaving a groove [31] for housing the branched segment [38] and the reversed portion [410] of the engaging member [400] in the pre-loaded condition.

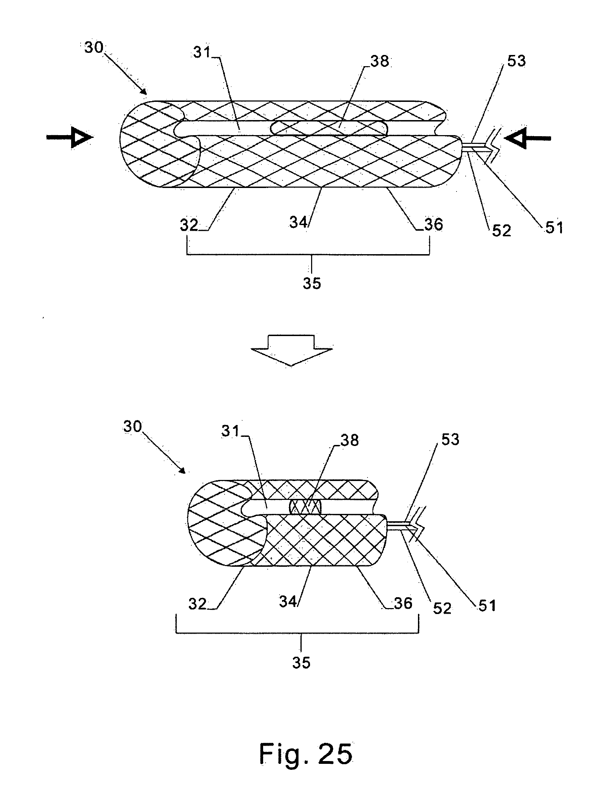

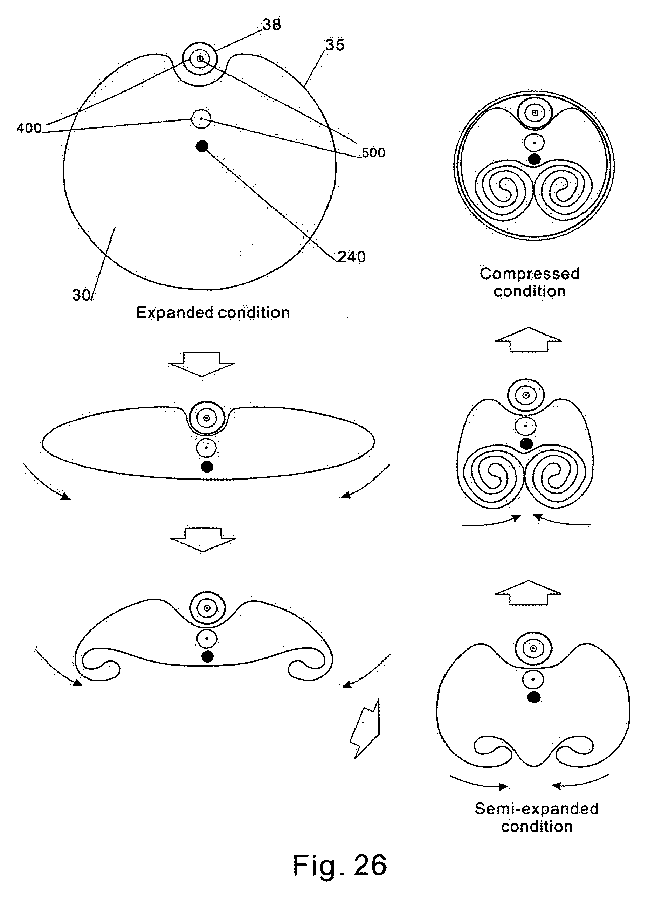

As illustrated in Fig. 25, by way of example, the branched stent graft [30] is first compressed in the axial direction to reduce the length. As illustrated in Fig. 26, the aortic segment [35] is then compressed vertically and along the radial axis into a flat shape. The two sides of the aortic segment [35] are then folded inwardly against each other and spirally toward the centre and away from the branched segment [38] on the top portion of the branched stent graft [30].

The aortic segment [35] is tied up by the first holding wire [51] at a semi-expanded condition before being folded further to the compressed condition. In the compressed condition, the aortic segment [35] is tied up by the second holding wire [52]. The branched segment [38] is tied up by the third holding wire [53].

The branched stent graft [30] is then disposed in the carrier member [300] to be attached with the inner core [240]. By the self-expandable nature, the branched stent graft [30] will acquire the circular shape of the lumen [302] of the carrier member [300].

Such folding manner ensures that the branched segment [38], which is at the top portion of the aortic segment [35], can be released and projected upward to the original shape immediately after the carrier member [300] is withdrawn and not being hindered by the folding of the aortic segment [35] in the compressed condition. Such folding manner also allows the upper portion of the aortic segment [35] containing the markings [18] in Figs. 9a and 9b to remain unfolded and stay on the top of the aortic segment [35] to appear clearly under the imaging equipment.

### Guiding Member

The elongated guiding member [500] is disposed in the engaging member [400] for guiding the engaging member into the aortic branch. The guiding member [500] includes an axially reversed portion at the distal end pointing in the proximal direction disposed in the reversed portion [410] of the engaging member [400] for facilitating the backward deployment of the branched segment [38], especially if the targeted aortic branch is in a downwardly pointing direction as shown in Fig. 5.

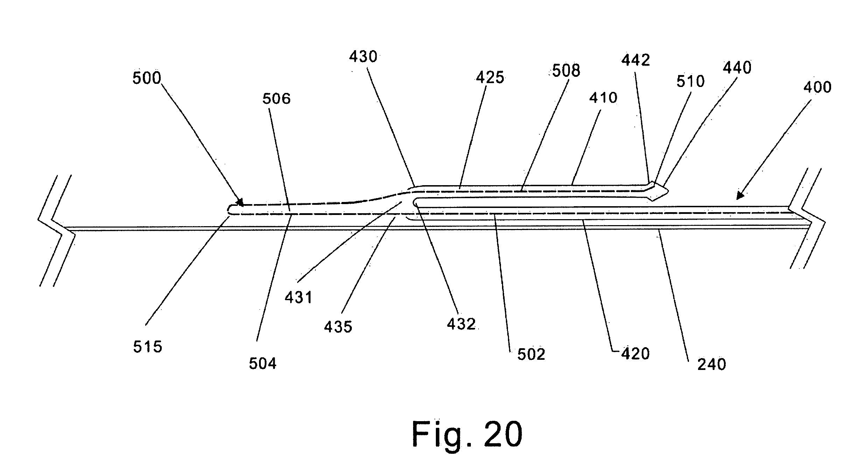

As illustrated in Figs. 20, 21, 23 and 24, the guiding member [500] may be a conventional hydrophilic guide wire being disposed in the engaging member [400]. By way of example, the diameter of the guiding member [500] is about 0.09 cm. The guiding member [500] extends out from the engaging member [400] through a hole [435] located at the turn portion [430] of the engaging member [400], and folds backward at a turning tip [515] to loop back into the engaging member [400] through the hole [435].

The guiding member [500] can be divided into four portions. An internal aortic portion [502] is disposed in the engaging member [400] before the hole [435]. An external aortic portion [504] is the portion distal to the internal aortic portion [502] and is located outside the engaging member [400] before folding backward. An external reversed portion [506] is the portion which folds backward and is located outside the engaging member [400] before entering back into the hole [435]. The external aortic portion [504] and the external reversed portion [506] of the guiding member [500] are disposed in the distal portion [32] of the branched stent graft [30] in the pre-loaded condition.

An internal reversed aortic portion [508] is the reversed portion which is located inside the engaging member [400] and ends before the tip [440] of the engaging member [400]. The internal reversed portion [508] ends as an angled tip [510] to better guide the guiding member [500] to enter into the aortic branch from the aorta.

### Middle core

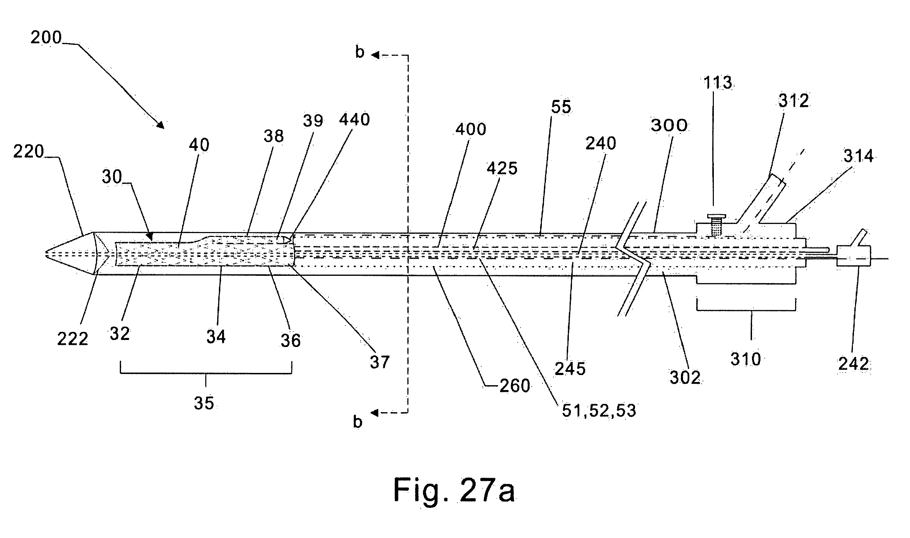

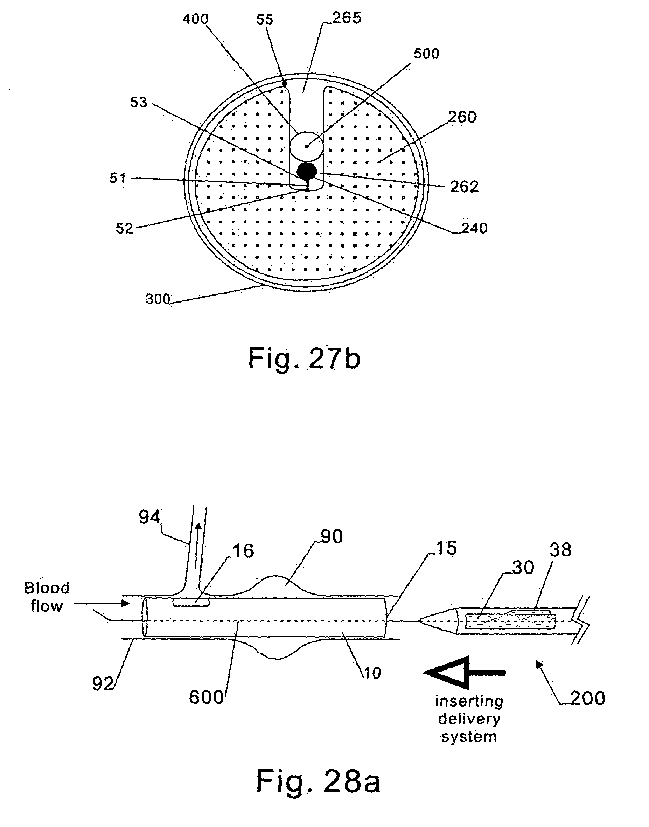

As shown in Figs. 27a and 27b, in another preferred embodiment, there is a middle core [260] disposed in the lumen [302] of the carrier member [300] terminating at the proximal end of the branched stent graft [30] preloaded in the delivery system [200]. A central lumen [262] extends throughout the central axis of the middle core [260] for receiving the inner core [240]. A slit [265] above the central lumen [262] extending throughout the middle core [260] receives the engaging member [400]. The middle core [260] prevents the branched stent graft [30] from moving backward when the carrier member [300] is pulled backward to release the branched stent graft [30], which enhances the performance of the delivery system [200].

In this preferred embodiment, the branched stent graft [30] is attached to the middle core [260] by the locking wire [55] instead of the inner core [240], so that it can be moved and rotated by manipulating the middle core [260]. The branched stent graft [30] may be detached from the middle core [260] by unscrewing the screw lock [113] at the proximal end and pulling and removing the locking wire [55].

### Delivery of branched stent graft

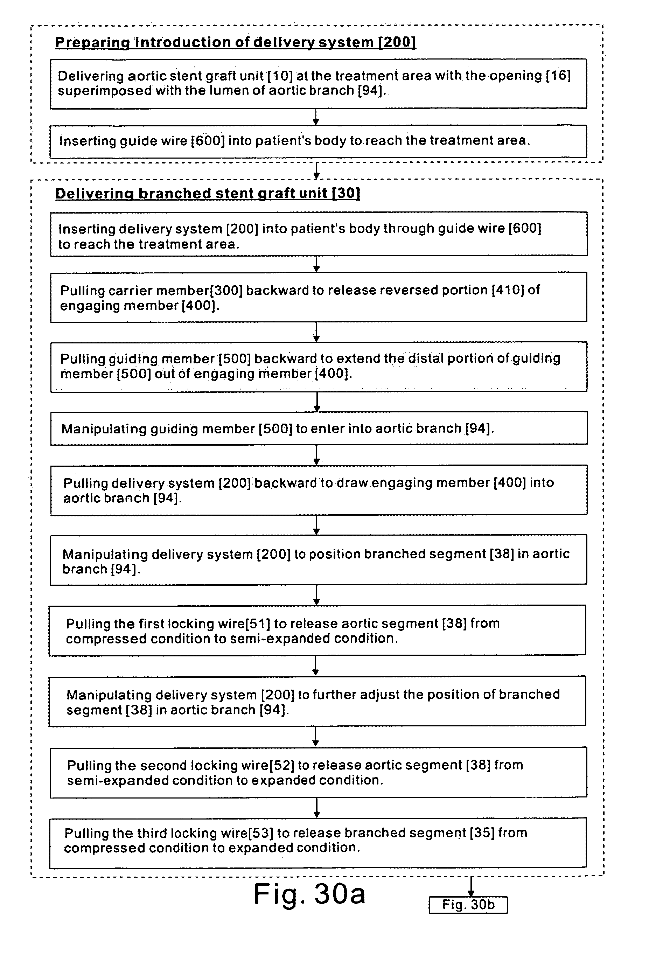

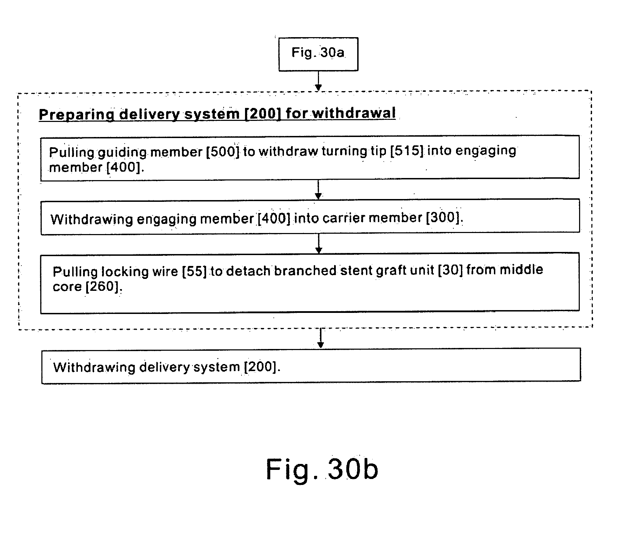

The delivery procedure of the branched stent graft [30] is illustrated in Figs. 30a and 30b. To deliver a branched stent graft [30] into human body, the location of the aortic stent graft [10] already placed at the aneurysm [90] and the aortic branch [94] to be stented are identified on the aortogram. Before inserting the delivery system [200], a guide wire [600] is first placed inside the patient's body for guiding the delivery system [200] to the treatment area.

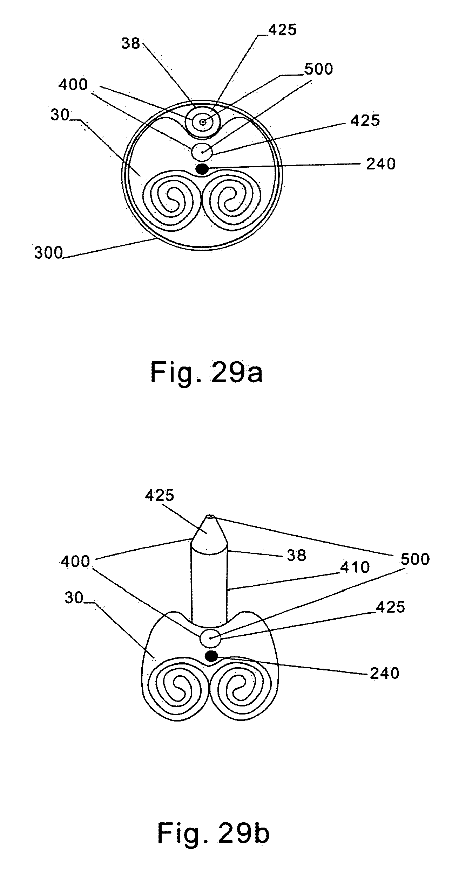

In this embodiment, the delivery system [200] includes the carrier member [300], the engaging member [400], the guiding member [500], the inner core [240] and the middle core [260]. As illustrated in Figs. 28a and 29a, the pre-assembled delivery system [200] is inserted through the guide wire [600] along the aorta [92] until the position of the branched segment [38] of the branched stent graft [30] is positioned in the lumen [15] of the aortic stent graft [10] distally beyond the aortic branch [94].

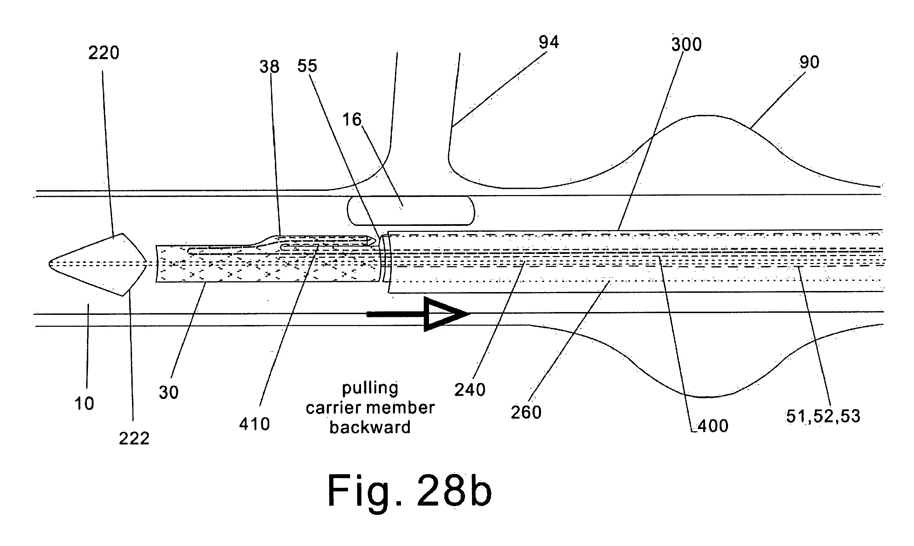

As illustrated in Figs. 28b and 29b, the carrier member [300] is then slidably pulled backward just to release the reversed portion [410] of the engaging member [400].

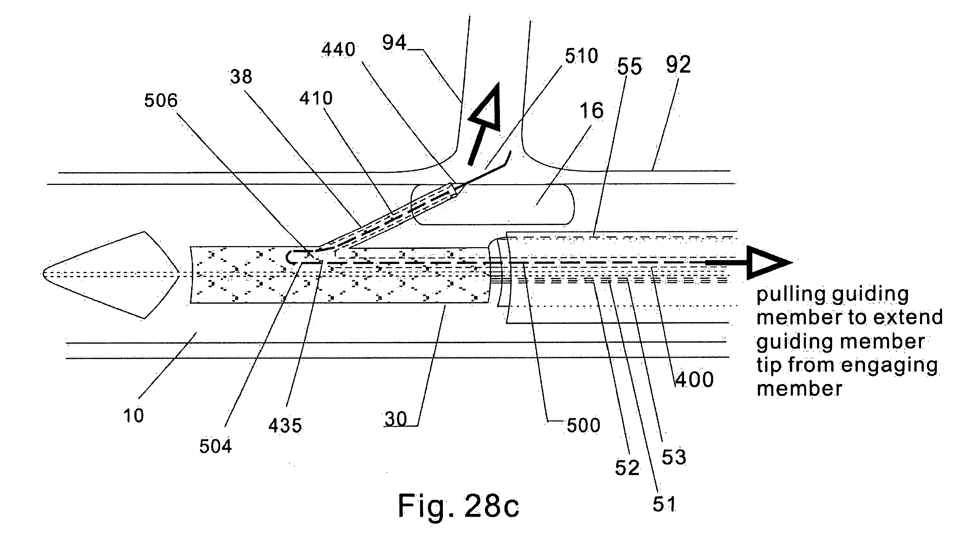

As illustrated in Fig. 28c, as the reversed portion [410] together with the branched segment [38] of the branched stent graft [30] begins to rise up in the released condition, the guiding member [500] is slidably pulled backward so that the external aortic portion [504] and the external reversed portion [506] are withdrawn into the hole [435] of the engaging member [400]. At the same time, the tip [510] of the guiding member extends out from the tip [440] of the engaging member [400].

The distal portion of the guiding member [500] is then manipulated to enter into the aortic branch [94] through the opening [16] of the aortic stent graft [10]. The angled tip [510] is turned to a position to make the guiding member [500] enter from the aorta [92] into the aortic branch [94]. The forward, backward and rotation movements of the guiding member [500] are controlled by a conventional torque device located at the end of the guiding member [500].

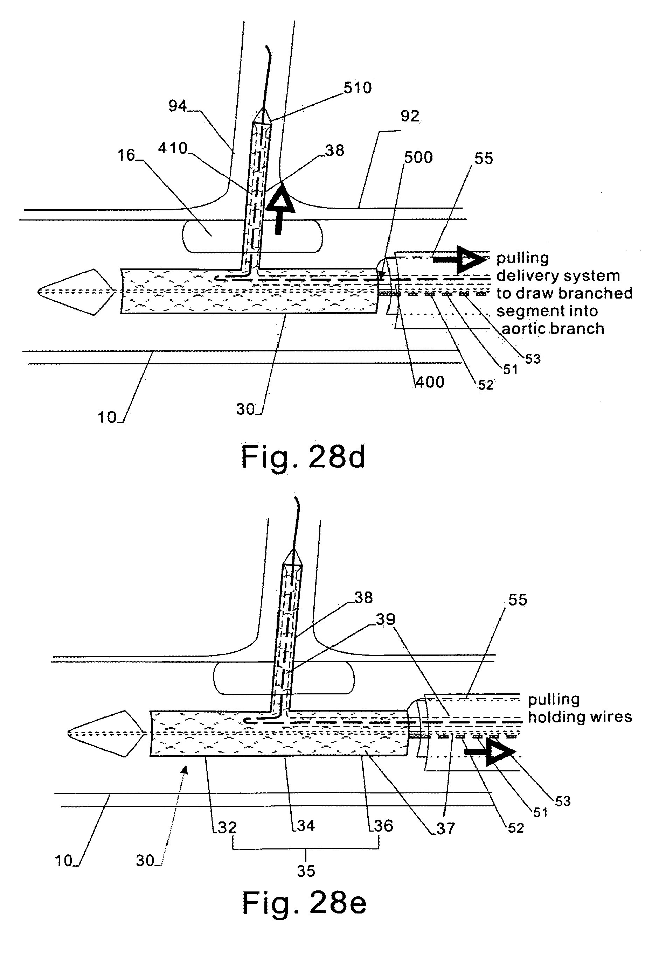

As illustrated in Fig. 28d, after the distal portion of the guiding member [500] has entered into the aortic branch [94], the delivery system [200] is then pulled backward so that the reversed portion [410] of the engaging member [400] loaded with the branched segment [38] of the branched stent graft [30] further enters into the aortic branch [94], guided by the distal portion of the guiding member [500]. The delivery system [200] is manipulated until the branched segment [38] loaded on the reversed portion [410] of the engaging member [400] is well positioned within the aortic branch [94].

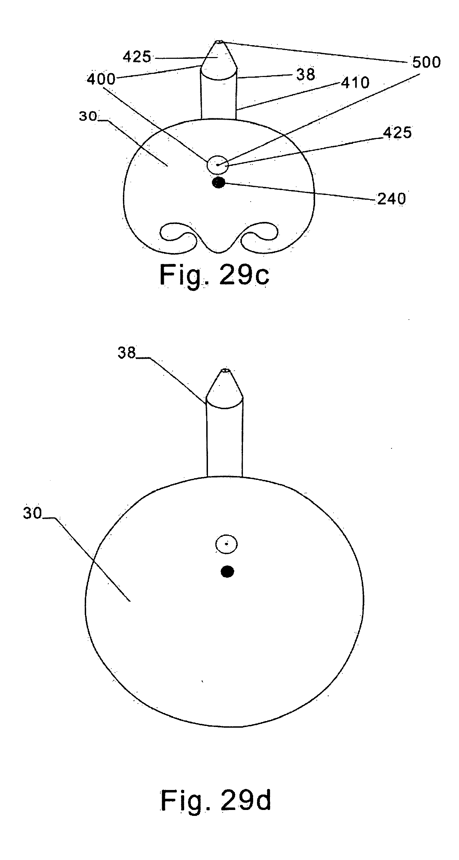

As illustrated in Figs. 28e and 29c, the first holding wire [51] that holds the aortic segment [35] of the branched stent graft [30] in the compressed condition is then pulled away to release the aortic segment [35] to the semi-expanded condition. The position of the branched segment [38] loaded on the reversed portion [410] of the engaging member [400] is then further adjusted by manipulating the delivery system [200] with the help of the radiopaque markings [46].

Thereafter, as illustrated in Fig. 29d, the second holding wire [52] that holds the aortic segment [35] is pulled away to release the aortic segment [35] from the semi-expanded condition to the expanded condition.

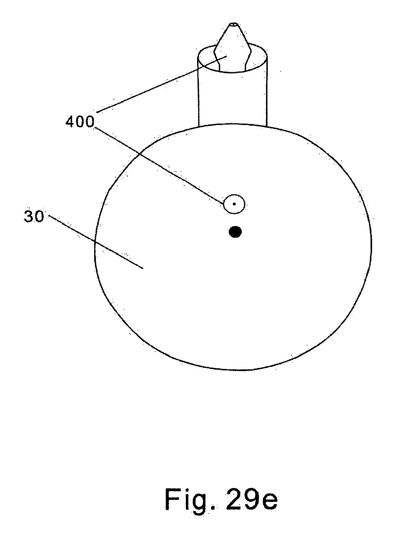

As illustrated in Fig. 29e, the branched segment [38] of the branched stent graft [30] is then released by pulling the third holding wire [53]. The branched stent graft [30] is now assembled with the aortic stent graft [10].

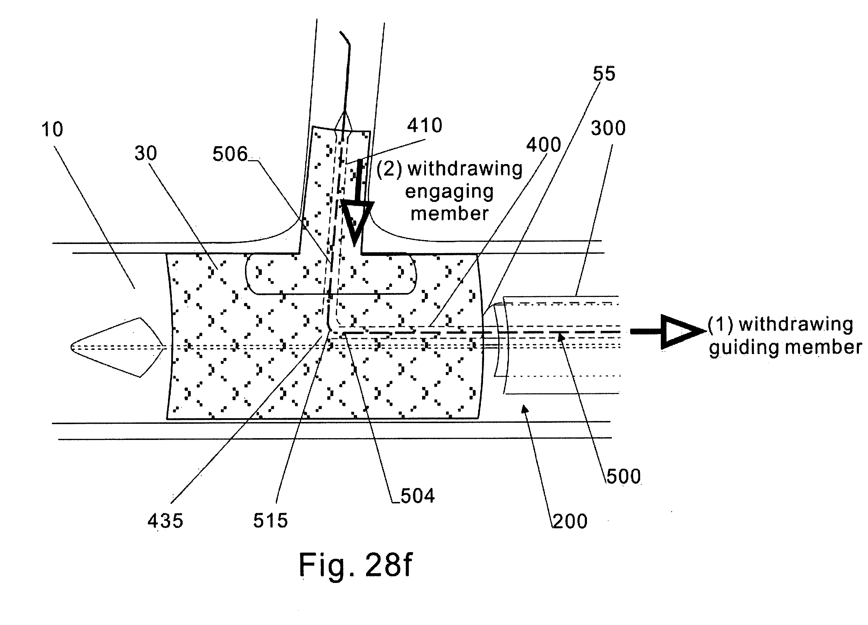

As illustrated in Fig. 28f, to withdraw the delivery system [200], the external aortic portion [504] and the external reversed portion [506] and the turning tip [515] of the guiding member [500] must first be totally withdrawn into the engaging member [400] through the hole [435]. The reversed portion [410] of the engaging member [400] is then slidably pulled backward to withdraw into the carrier member [300].

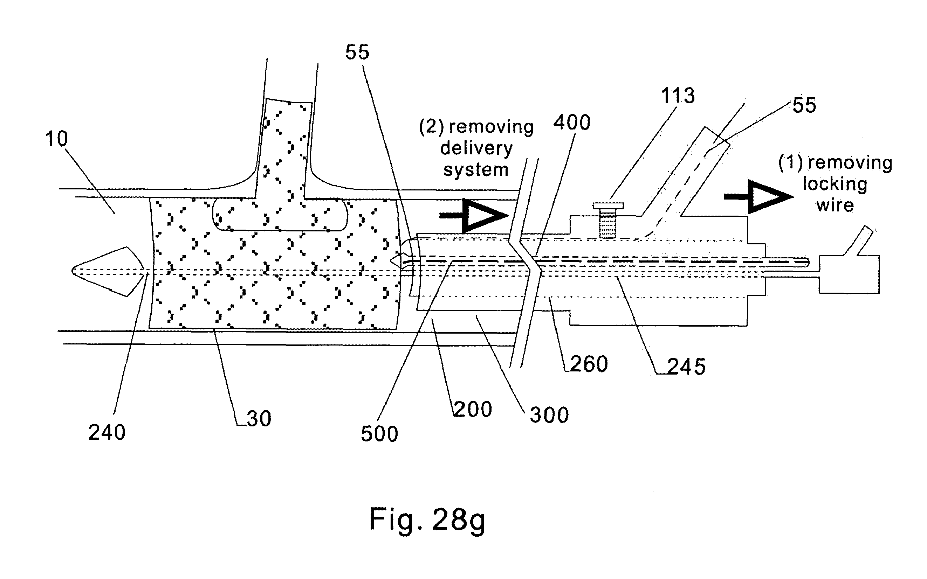

Thereafter, as illustrated in Fig. 28g, the locking wire [55] attaching the branched stent graft [30] to the middle core [260] is removed by unscrewing the screw lock [113] to detach the branched stent graft [30] from the delivery system [200]. The delivery system [200] is then withdrawn from human body.

While the invention has been described in detail with reference to disclosed embodiments, various modifications within the scope of the invention will be apparent to those of ordinary skill in this technological field. It is to be appreciated that features described with respect to one embodiment typically may be applied to other embodiments.

## Claims

1. An aortic stent graft expandable between a compressed condition and an expanded condition for use with a branched stent graft in treatment of an aneurysm, having a stent graft comprising a lumen extending therethrough, **characterized in that**:
the lumen is configured for receiving an aortic segment of said branched stent graft in the expanded condition, and
the stent draft comprises at least one axial opening for receiving a branched segment of said branched stent graft,
whereby said opening is superimposed on the aortic segment and the branched segment of said branched stent graft when assembled.

2. The aortic stent graft, as recited in claim 1, further comprises radiopaque markings spaced apart thereon in axis with said opening in the expanded condition, for indicating the orientation of the opening of said aortic stent graft.

3. The aortic stent graft, as recited in claim 2, wherein said markings appear outside of said aortic stent graft in the compressed condition.

4. A branched stent graft, expandable between a compressed condition and an expanded condition, for use with an aortic stent graft in treatment of an aneurysm, comprises an aortic segment for disposing in the lumen of said aortic graft stent, having a distal portion, a proximal portion, a middle portion therebetween, and a main lumen extending therethrough, **characterized in that**, it further comprises:
a branched segment extending from the middle portion of said aortic segment for disposing through the opening of said aortic stent graft, having a connecting end and an open end, and a branch lumen extending therethrough,
wherein the main lumen of the aortic segment connecting with the branch lumen of the branched segment at said connecting end.

5. The branched stent graft, as recited in claim 4, wherein said aortic segment further comprises radiopaque markings spaced apart thereon in axis with said connecting end in the expanded condition, for indicating the orientation of said aortic segment.

6. The branched stent graft, as recited in claim 4, wherein said branched segment further comprises radiopaque marking thereon for indicating the orientation of said branched segment.

7. The branched stent graft, as recited in claim 4, wherein the proximal portion of said aortic segment is configured and sized for disposing into a distal portion of an aortic segment of another branched stent graft in the expanded condition.

8. The branched stent graft, as recited in claim 4, wherein the distal portion of said aortic segment is configured and sized for receiving a proximal portion of an aortic segment of another branched stent graft in the expanded condition.

9. The branched stent graft, as recited in claim 4, wherein said aortic segment is partially grafted, having a non-grafted portion for associating with a second opening of an aortic stent graft and a branched segment of a second said partially grafted branched stent graft,
whereby said non-grafted portion allows blood access through the second opening of the aortic stent graft and the branched segment of the second branched stent graft.

10. The branched stent graft, as recited in claim 4, wherein said aortic segment forms an angle with said branched segment of approximately 40 to 60 degrees.

11. The branched stent graft, as recited in claim 4, wherein said branched stent graft is further expandable between the compressed condition and a semi-expanded condition and between the semi-expanded condition and the expanded condition,
whereby in the semi-expanded condition, the position of the branched segment of said branched stent graft is adjusted in an aortic branch, before expanding to the expanded condition.

12. A delivery system for delivering a branched stent graft, comprising:
an elongated carrier member for carrying said branched stent graft to a target location, having a lumen extending therethrough for receiving an elongated engaging member, a distal end, and a distal portion for receiving said branched stent graft in the compressed condition following said distal end of the carrier member; and
an elongated engaging member disposed' in and axially slidable relative to said carrier member for engaging with said branched stent graft, operating between a pre-loaded condition and a released condition, having a lumen extending therethrough,
**characterized in that** the elongated engaging member further comprises:
a distal end axially reversed to point in a proximal direction,
an axially reversed portion for engaging with the branched segment of said branched stent graft in the pre-loaded condition following said distal end of the engaging member,
a turning portion having a concave side and a convex side thereon following said reversed portion of the engaging member, and
an aortic portion following said turning portion for engaging with the aortic segment of said branched stent graft in the pre-loaded condition,
whereby, the engaging member is released from the carrier member to the released condition, the reversed portion of the engaging member deploys the branched segment of the branched stent graft into an aortic branch in a backward manner.

13. The delivery system as recited in claim 12, wherein said carrier member is an outer sheath.

14. The delivery system as recited in claim 12, wherein said engaging member is a catheter.

15. The delivery system as recited in claim 12, further comprises:
a hole formed on the convex side of the turning portion of said engaging member,
a guiding member disposed in and axially slidable relative to said engaging member, having:
a distal end disposed in the reversed portion of said engaging member,
an axially reversed portion following said distal end of the guiding member partially disposed in the reversed portion of the engaging member and partially disposed outside the engaging member through said hole on the turning portion of the engaging member in the pre-loaded condition,
whereby in the released condition of the engaging member, the reversed portion of the guiding member disposed outside the engaging member is slidably pulled into the hole, the distal end of the guiding member is slidably pushed out the distal end of the engaging member and guides the engaging member into the aortic branch in a backward manner.

16. The delivery system as recited in claim 15, wherein said guiding member is a guide wire.

17. The delivery system as recited in claim 12, further comprises:
an elongated inner core disposed in said carrier member, slidable relative thereto, having a distal end terminating at the distal end of said carrier member, and a tapered tip connected to said distal end of said inner core.

18. The delivery system as recited in claim 17, further comprises:
an elongated middle core disposed in said carrier member, slidable relative thereto and terminating before said branched stent graft in the distal portion of said carrier member, having an axial slit in said middle core for receiving said engaging member and said inner core,
whereby said middle core prevents the branched stent graft from moving backward when said carrier member is pulled backward to release the branched stent graft.

19. The delivery system as recited in claim 12, wherein said engaging member forms a tapered tip at the distal end of said engaging member.

20. The delivery system as recited in claim 19, wherein said tapered tip further forms a back portion tapering in an opposite direction with respect to the tapered tip.

21. The delivery system as recited in claim 17, wherein said aortic segment of said branched stent graft is removably coupled to said inner core.

22. The delivery system as recited in claim 18, wherein said aortic segment of said branched stent graft is removably coupled to said middle core.

23. The delivery system as recited in claim 12, wherein said branched stent graft in the compressed condition forms an indented cross-sectional shape disposed uniformly along said middle and proximal portions, forming a groove for disposing the branched segment of said branched stent graft.

24. The delivery system as recited in claim 12, wherein said carrier member further comprises markings outside the distal portion of said carrier member, for indicating the orientation of said branched stent graft inside said carrier member.

25. A method for folding an aortic segment of a branched stent graft from an expanded condition to a compressed condition, comprising the steps of:
(a) compressing the aortic segment along the radial axis into a substantially flat condition, wherein the branched segment is disposed in the middle position,
(b) folding the two sides of the aortic segment in said flat condition spirally and inwardly against each other, whereby in the compressed condition, the branched segment remains uncovered by the aortic segment and the expansion of the branched segment is not hindered by said folded aortic segment.

26. The method as recited in claim 25, prior to step (a), further comprises the step of:
compressing the aortic segment along the longitudinal axis.

27. A modular stent graft set, comprising at least one aortic stent graft of claim 1 and at least one branched stent graft of claim 4.

28. The modular stent graft set, as recited in claim 27, wherein said aortic stent grafts are provided with variation in at least one of the following dimensions:
the length of said distal portion,
the length of said proximal portion,
the length of said opening,
the diameter of said distal portion, and
the diameter of said proximal portion.

29. The modular stent graft set, as recited in claim 27, wherein said branched stent grafts are provided with variation in at least one of the following dimensions:
the length of said distal portion,
the length of said proximal portion,
the length of said branched segment,
the diameter of said distal portion,
the diameter of said proximal portion, and
the diameter of said branched segment.
